# EUROPEAN PATENT SPECIFICATION

(11) **EP 1 511 481 B1**
(45) Date of publication and mention of the grant of the patent: **27.10.2010**
(21) Application number: 03730122.3
(22) Date of filing: 27.05.2003
(51) Int. Cl.: A61K 31/44, A61P 27/02

(54) **OPHTHALMOLOGICAL USE OF ROFLUMILAST FOR THE TREATMENT OF DISEASES OF THE EYE**
OPHTHALMOLOGISCHE ZUBEREITUNG ENTHALTEND ROFLUMILAST IN DER BEHANDLUNG VON KRANKHEITEN DER AUGEN
UTILISATION OPHTALMOLOGIQUE DE DU ROFLUMILAST POUR LE TRAITEMENT DE MALADIES DE L'OEIL

(30) Priority: 28.05.2002 EP 02011830; 28.05.2002 DE 10223828; 14.03.2003 DE 10311613
(43) Date of publication of application: 09.03.2005
(73) Proprietor: Nycomed GmbH, 78467 Konstanz (DE)
(72) Inventor: KOENEN, Rüdiger, 78464 Konstanz (DE); LINDER, Rudolf, 78464 Konstanz (DE)
(74) Representative: Kratzer, Bernd
(86) International application number: PCT/EP2003/005536
(87) International publication number: WO 2003/099278

(56) References cited:
- EP-A- 1 118 615
- WO-A-00/18388
- WO-A-01/32165
- WO-A-01/57025
- WO-A-01/90076
- WO-A-95/01338
- US-A- 4 753 945
- US-A- 5 011 843

## Description

### Technical field

The present invention relates to the use of Roflumilast, the N-oxide thereof or salts thereof for the manufacture of a pharmaceutical preparation for treatment of diseases of the eye, wherein the disease of the eye is dry eye syndrome (keratitis sicca). In addition, the present invention relates to an ophthalmological pharmaceutical preparation in the form of oily eye drops comprising a therapeutically effective and suitable amount of the above active ingredient, wherein the preparation comprises medium chain length triglycerides.

### Prior art

Cyclic nucleotide phosphodiesterase (PDE) inhibitors (specifically of type 4) are currently of special interest as a new generation of active ingredients for treating inflammatory disorders, especially disorders of the airways such as asthma or airway obstructions (such as, for example, COPD = chronic obstructive pulmonary disease). A number of PDE 4 inhibitors are currently undergoing advanced clinical testing, including a dosage form for oral administration comprising the active ingredient N-(3,5-dichloropyrid-4-yl)-3-cyclopropylmethoxy-4-difluoromethoxybenzamide (INN: roflumilast). This and other compounds with a benzamide structure and their use as cyclic nucleotide phosphodiesterase (PDE) inhibitors for the treatment of diseases are described in WO 95/01338.

US-A-5 011 843 discloses a method of lowering intraocular pressure in a mammal which comprises administering to the eye of said mammal a pharmaceutical composition comprising a therapeutically effective amount of a phosphodiesterase inhibitor. In particular, the use of a phosphodiesterase inhibitor, especially those increasing cAMP levels, for the manufacture of eye drops or ointments for the treatment of glaucoma is disclosed.

### Description of the invention

Surprisingly it has now been found out, that pharmaceutical preparations comprising the PDE 4 inhibitor roflumilast show a very good effect and other beneficial properties in the treatment of diseases of the eye.

In one aspect the present invention is therefore related to the use of a compound selected from the group consisting of roflumilast, salts of roflumilast, the N-oxide of the pyridine residue of roflumilast or salts thereof for the manufacture of a pharmaceutical preparation for the prevention or treatment of a disease of the eye., wherein the disease of the eye is dry eye syndrome (keratitis sicca).

Roflumilast is the INN for a compound of the formula I 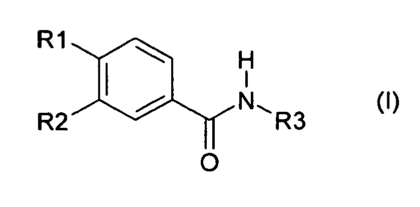 in which
- R1: is difluoromethoxy,
- R2: is cyclopropylmethoxy and
- R3: is 3,5-dichloropyrid-4-yl.

This compound has the chemical name N-(3,5-dichloropyrid-4-yl)-3-cyclopropylmethoxy-4-difluoromethoxybenzamide (INN: roflumilast). The N-oxide of roflumilast has the chemical name 3-cyclopropylmethoxy-4-difluoromethoxy-N-(3,5-dichloropyrid-4-yl 1-oxide)benzamide.

This compound of the formula I, its salts, the N-oxide, its salts and the use of these compounds as phosphodiesterase (PDE) 4 inhibitors are described in the international patent application WO 95/01338.

Salts suitable for compounds of the formula I - depending on the substitution - are all acid addition salts but, in particular, all salts with bases. Particular mention may be made of the pharmacologically acceptable salts of the inorganic and organic acids and bases normally used in pharmaceutical technology. Pharmacologically unacceptable salts, which for example, may be the initial products of the process for preparing the compounds of the invention on the industrial scale are converted into pharmacologically acceptable salts by processes known to the skilled worker. Those suitable on the one hand are water-soluble and water-insoluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)benzoic acid, butyric acid, sulphosalicylic acid, maleic acid, lauric acid, malic acid, fumaric acid, succinic acid, oxalic acid, tartaric acid, embonic acid, stearic acid, toluenesulphonic acid, methanesulphonic acid, or 3-hydroxy-2-naphthoic acid, the acids being employed to prepare the salts in the equimolar ratio of amounts, or one differing therefrom - depending on whether the acid is monobasic or polybasic and depending on which salt is desired.

On the other hand, salts with bases are also particularly suitable. Examples of basic salts which may be mentioned are lithium, sodium, potassium, calcium, aluminium, magnesium, titanium, ammonium, meglumine or guanidinium salts, once again the bases being employed to prepare the salts in the equimolar ratio of amounts or one differing therefrom.

The pharmaceutical preparations comprising roflumilast, salts of roflumilast, the N-oxide of the pyridine residue of roflumilast or salts thereof for treatment of diseases of the eye can be prepared by processes, which are known per se and familiar to the person skilled in the art. As pharmaceutical preparations, the active ingredient according to the invention can be either employed as such, or preferably in combination with suitable pharmaceutical auxiliaries, e.g. in the form of tablets, coated tablets, capsules, suppositories, patches, emulsions, suspensions, gels or solutions, the active compound content advantageously being between 0.1 and 95%. The person skilled in the art is familiar with auxiliaries, which are suitable for the desired pharmaceutical preparations on account of his expert knowledge. In addition to solvents, gel formers, ointment bases and other active compound excipients, for example antioxidants, dispersants, emulsifiers, preservatives, solubilizers or permeation promoters, can be used. Further examples, which may be mentioned, are carriers and/or excipients which are suitable for producing tablets, emulsions, suspensions, sprays, oils, ointments, greasy ointments, creams, pastes, gels, foams or solutions, and transdermal therapeutic systems.

In a preferred embodiment according to the invention the pharmaceutical preparation for treatment of diseases of the eye is an ophthalmological pharmaceutical preparation suitable for administration in, on or close to the eye.

In another embodiment the pharmaceutical preparation for treatment of diseases of the eye according to the invention is an administration form for systemic application.

Another subject of the invention is therefore an ophthalmological pharmaceutical preparation in the form of oily eye drops comprising a therapeutically effective and pharmacologically suitable amount of an active pharmaceutical ingredient selected from the group of compounds roflumilast, salts of roflumilast, the N-oxide of roflumilast and salts together with one or more pharmaceutically acceptable carriers and/or excipients, wherein the pharmaceutical preparation comprises medium chain length triglycerides.

Oily eye drops comprise according to the invention oily suspensions of the active ingredient.

It is preferred in this connection for the particle size of the active ingredient employed to be 90% less than 10 µm.

Preferably used in the case of aqueous suspensions are suspension stabilizers such as, for example, substituted celluloses (e.g. methylcellulose, hydroxypropylmethylcellulose), polyvinyl alcohol, polyvinylpyrrolidone, in addition to preservatives (e.g. chlorocresol, phenylmercury compounds, phenylethanol, benzalkonium chloride or mixtures of individual components) and, where appropriate, sodium chloride to adjust to isotonicity. Preferably employed according to the invention in the case of oily eye drops are castor oil, peanut oil or medium chain length triglycerides. It is possible in the case of eye ointments to use according to the invention ointment bases which have the following properties: sterility or extremely low microbe content, non-irritating, good activity, good distribution of the active ingredient or its solution in the ointment, suppleness, rapid dispersion as fine film over the eyeball, good adhesion to the eye, good stability and low impairment of vision. Hydrocarbon- or cholesterol-containing bases will therefore preferably be employed according to the invention for eye ointments. In the case of petrolatum, liquid paraffin is preferably added for consistency reasons. To achieve good spreading, it is preferred according to the invention to provide compositions of limited viscosity. The viscosity at 32°C is preferably below 1 000 mPa.s, and the yield point is preferably below 300 mPa. In the case of suspension ointments it is preferred according to the invention for 90% of the active ingredient particles to be below 10 µm, and no particles above 90 µm should occur. In the case of water/oil emulsion ointments, it is preferred according to the invention to add preservatives such as benzalkonium chloride, thiomersal or phenylethyl alcohol.

The pharmaceutical preparation of the invention for systemic application can be a transdermal therapeutic system (TTS), for example a system as described in Pharmazeutische Technologie: Moderne Arzneiformen, Wissenschaftliche Verlagsgesellschaft mbH Stuttgart 1997, pages 81 et sec. TTSs are characterized in principle by a defined supply of medicinal substance to the skin, a total dose of the medicinal substance in the TTS, a total area and an area which is possibly different therefrom for release of the medicinal substance, a covering sheet (backing layer) which is impermeable to the medicinal substance, a medicinal substance reservoir, a control element which controls the supply of medicinal substance to the skin, a (pressure-sensitive) adhesive layer and a detachable protective layer. It is possible on occasions for more than one function to be fulfilled by one and the same element, e.g. reservoir, control and adhesive functions by a suitable adhesive matrix. From the viewpoint of pharmaceutical technology, TTSs are categorized according to the way the control function is achieved, that is to say how it controls the supply of medicinal substance to the skin. Examples, which are mentioned here are TTSs with membrane permeation-controlled release (membrane moderated drug delivery), TTSs with matrix diffusion-controlled release and TTSs with microreservoir solution-controlled release.

TTSs with membrane permeation-controlled release are characterized by a polymer membrane composed of a PVA-VA copolymer (Chronomer®) which controls the permeation of the medicinal substance from the reservoir into the skin. The medicinal substance is initially in the form of solid particles or as a dispersion or solution in the reservoir. The polymer membrane can be attached to the reservoir in various ways (extrusion, encapsulation, microencapsulation). TTSs with matrix diffusion-controlled release have a comparatively simpler structure. They contain no separate control element The release of medicinal substance is controlled by a lipophilic or hydrophilic polymer matrix and/or the adhesive layer. It is possible to distinguish, according to the characteristics of the matrix, between TTSs with a matrix in gel form and TTSs which represent solid polymer laminates. The medicinal substance reservoir is formed by the medicinal substance dissolved in the matrix (monolithic system) or a homogeneous dispersion of solid medicinal substance particles. A matrix TTS can be produced by mixing the medicinal substance particles with a viscous liquid or semisolid polymer at room temperature, followed by crosslinking the polymer chains. A further possibility is also to mix the medicinal substance at elevated temperature with softened polymer (hot melt technique), or the two components (dissolved in an organic solvent) are mixed together and the solvent is then removed in vacuo (solvent evaporation). Shaping is possible by pouring into suitable moulds, spreading with special devices (knives) or by extrusion. In the case of TTSs with microreservoir solution-controlled release (microsealed drug delivery, MDD principle), numerous microcompartments containing the active ingredient and 10-200 µm in size are embedded in a matrix which represents both reservoir and delivery-control element. Because of the matrix, these TTSs are actually assigned to the matrix systems. For production, the medicinal substance is initially dispersed together with water and 40% polyethylene glycol 400 in isopropyl palmitate, which acts as permeation promoter. The resulting dispersion is incorporated by using a special high-energy dispersion technique into a viscous silicone elastomer which simultaneously undergoes catalytic polymerization. The medicinal substance-containing matrix can be shaped specifically by melt or extrusion techniques before it is combined with the carrier in the manner already described. Depending on the physicochemical properties of the medicinal substances and the intended liberation, it is possible to cover the matrix with a layer of a biocompatible polymer in order thus to modify the mechanism and the rate of liberation.

In another embodiment of the invention the pharmaceutical preparation for systemic administration is a dosage form for oral administration, preferably a tablet.

Suitable pharmaceutical excipients, which may be used in the dosage form for oral administration of the invention are pharmaceutical excipients such as fillers, additional binders, tablet disintegrants or else lubricants and release agents. Other suitable excipients, which may be present are, for example, flavoring substances (such as flavors and sweeteners), buffer substances, preservatives, coloring substances (such as iron oxid yellow or red) or else emulsifiers. Flavors are usually added in a proportion of from 0.05 to 1 % by weight. Other flavoring substances by way of example are acids such as citric acid, sweeteners such as saccharin, aspartame, cyclamate sodium or maltol, which are added according to the desired result.

In a preferred embodiment of the invention the tablet for oral administration is employing polyvinylpyrrolidone (PVP) as binder. The polyvinylpyrrolidone (PVP) employed according to the invention is, in particular, a water-soluble PVP with an average molecular weight above 2 000, preferably above 20 000. Examples, which may be mentioned are Kollidon 12 PF (molecular weight 2 000-3 000), Kollidon 17 PF (molecular weight 7 000-11 000), Kollidon 25 (molecular weight 28 000-34 000), Kollidon 30 (molecular weight 44 000-54 000), Kollidon 90 F (molecular weight 1 000 000-1 500 000). PVP of higher molecular weight such as, for example, Kollidon 25, Kollidon 30 and Kollidon 90 F may be mentioned as preferred.

It is possible if desired to employ in addition to PVP other binders such as polyvinyl acetate (e.g. Kollidon® VA 64), gelatin, com starch mucilage, preswollen starches (Starch 1500), hydroxypropylmethylcellulose (HPMC) or hydroxypropylcellulose (L-HPC).

Fillers suitable according to the invention are fillers such as calcium carbonate (e.g. MagGran® CC or Oestab® 95) and sodium carbonate, sugar alcohols such as mannitol (e.g. Perlitol® or Parteck® M), sorbitol (e.g. Karion®), xylitol or maltitol, starches such as com starch, potato starch and wheat starch, microcrystalline cellulose, saccharides such as glucose, lactose (e.g. lactose monohydrate), levulose, sucrose and dextrose. It is also possible if desired to use mixtures thereof. Corn starch, microcrystalline cellulose and lactose may be mentioned as preferred.

Examples of suitable lubricants and release agents, which may be mentioned are sodium stearyl fumarate, magnesium stearate, calcium stearate, stearic acid, talc and colloidal anhydrous silica (Aerosil).

Disintegrants suitable according to the invention are, in particular, insoluble polyvinylpyrrolidone (insoluble PVP, crospovidone), carboxymethylstarch sodium [= sodium starch glycolate], sodium carboxymethylcellulose, alginic acid, and starches able to carry out the function of a disintegrant (e.g. Starch 1500).

The proportion (in percent by weight based on the finished dosage form) of PDE 4 inhibitor in the dosage form of the invention is usually, depending on the nature of the PDE 4 inhibitor, from 0.01 to 50% by weight. The proportion of PDE 4 inhibitor is preferably up to 20% by weight.

The proportion (in percent by weight based on the finished dosage form) of binder (PVP and, where appropriate, other binders) may preferably be according to the invention from 0.5 to 20% by weight. The proportion of PVP is preferably from 1 to 5% by weight, particularly preferably 2 to 3% by weight.

The proportion (in percent by weight based on the finished dosage form) of filler in the tablet of the invention is advantageously from 40 to 99% by weight. The proportion of filler is preferably from 60 to 97% by weight.

The proportion (in percent by weight based on the finished dosage form) of disintegrant in the rapidly disintegrating tablet can usually be up to 35% by weight. The proportion of disintegrant is preferably from 2 to 20% by weight. The proportion of disintegrant is particularly preferably from 5 to 10% by weight.

The proportion (in percent by weight based on the finished dosage form) of lubricant or release agent in the rapidly disintegrating tablet is usually from 0.1 to 5% by weight. The proportion of lubricant or release agent is preferably from 0.3 to 3% by weight. The proportion of lubricant or release agent is particularly preferably from 0.5 to 2% by weight.

In a preferred embodiment of the invention, the dosage form is a tablet. It is preferred for the tablet, besides the active ingredient and PVP, to comprise as further pharmaceutical excipients at least one filler and at least one lubricant or release agent.

The pharmaceutical preparation of the invention can be produced by processes for producing tablets and pellets, which are known to the skilled worker.

In one embodiment of the invention, the pharmaceutical preparation of the invention is produced by producing a solid solution of the active ingredient in the binder PVP as carrier. This can take place for example by the solvent method in which PVP, the active ingredient and, where appropriate, other pharmaceutical excipients are dissolved in a suitable solvent, and the solvent is subsequently removed again by spray drying, normal drying, vacuum drying or freeze-drying. It has been found, surprisingly, that production of the solid solution is also possible by the mixing method in which the active ingredient and, where appropriate, other pharmaceutical excipients are vigorously mixed together with PVP.

In the event of further processing of a solid solution to tablets or pellets, the solid solution may be processed as active ingredient component together with the filler, binder, disintegrant and lubricant components by production processes familiar to the skilled worker to give the oral dosage forms of the invention. A solid solution of the active ingredient in the binder PVP as carrier means according to the invention a solid solution with amorphous structure in which the active ingredient is in the form of a molecular dispersion in the carrier material.

The pharmaceutical preparation can be produced by a process for producing a dosage form in tablet or pellet form for oral administration of the active ingredient, comprising the steps: (a) production of an active ingredient preparation in the form of a solid solution in PVP of the active ingredient, (b) production of a mixture of an active ingredient preparation and pharmaceutical excipients and (c) granulation of the mixture obtained in (b) with an aqueous solution of PVP.

In the case of dosage forms of the invention in the form of tablets, the granules obtained in (c) can, after drying and mixing with lubricants or release agents, be compressed in a tablet press. In the case of dosage forms of the invention in the form of pellets, the wet granules obtained in (c) can be processed by the extruder/spheroidizer process to suitable pellets. Alternatively, dispersions/suspensions of an active ingredient preparation can be applied in the form of a solid solution in PVP of the active Ingredient in a suitable solvent to pellet-like carriers (e.g. nonpareils or HPMC-containing pellets).

The dosage form of the invention can also be produced by granulating a mixture of active ingredient and pharmaceutical excipients with an aqueous PVP solution, drying the granules and, if desired, admixing other pharmaceutical excipients. Wet preparations obtained after granulation can then be further processed to pellets and can subsequently be packed into capsules. Dried granules can - if desired after admixture of other pharmaceutical excipients - after mixing with a release agent be compressed in a tablet press. The granulation preferably takes place in a fluidized bed granulator under suitable conditions. It is moreover possible if desired for the active ingredient to be admixed to the other pharmaceutical excipients in the form of a trituration with a pharmaceutical excipient (especially a filler). This is particularly preferred when the active ingredient content in the dosage form is less than 5% by weight. Such a trituration can normally be obtained by grinding the active ingredient with a pharmaceutical excipient (especially a filler).

The pharmaceutical preparation can therefore also be produced by a process for producing a dosage form in tablet or pellet form for oral administration of the active ingredient comprising the steps:
(a) production of a mixture of active ingredient and pharmaceutical excipients and
(b) granulation of the mixture obtained in (a) with an aqueous solution of PVP.

The pharmaceutical preparation can also be produced by granulation of a mixture of
(a) active ingredient, or a trituration of the active ingredient with com starch,
(b) com starch and
(c) lactose monohydrate
with an aqueous PVP solution, drying of the granules, mixing of the granules with a release agent and compression in a tablet press.

Alternatively, the pharmaceutical preparation can be produced by granulation of a mixture of
(a) active ingredient, or a trituration of the active ingredient with com starch,
(b) com starch,
(c) microcrystalline cellulose and
(d) sodium carboxymethylstarch
with an aqueous PVP solution, drying of the granules, mixing of the granules with a release agent and compression in a tablet press.

The pharmaceutical preparation can be produced by granulation of a mixture of pharmaceutical excipients with a suspension of the active ingredient in an aqueous PVP solution, drying of the granules and, if desired, admixture of further pharmaceutical excipients. The preparations obtained in this way can then, after mixing with a release agent, be compressed in a tablet press. The granulation preferably takes place in a fluidized bed granulator under suitable conditions.

The pharmaceutical preparation can also be produced by a process comprising the steps:
(a) production of a mixture of pharmaceutical excipients and
(b) granulation of the mixture obtained in (a) with a suspension of the active ingredient in an aqueous solution of PVP.

The pharmaceutical preparation can be produced by granulation of a mixture of com starch and lactose monohydrate with a suspension of the active ingredient in an aqueous solution of PVP, drying of the granules, mixing of the granules with a release agent and compression in a tablet press.

The production of pharmaceutical preparation according to the invention is described by way of example below. The following examples explain the invention in more detail.

### Examples

### Production of pharmaceutical preparations of the invention

### Example 1

### Composition of an eye ointment (quantity for 1 000 grams)

| | |
|---|---|
| Roflumilast | 1 g |
| Cetyl alcohol | 4 g |
| High-viscosity paraffin | 200 g |
| White petrolatum | 795 g |

Production: A clear melt of the cetyl alcohol, the high-viscosity paraffin and the white petrolatum is prepared at about 70°C. The micronized roflumilast (90% of the particles below 10 µm) is stirred in, and a homogeneous dispersion is prepared using an Ultra-Turrax. The suspension is cooled to room temperature while stirring and used to fill suitable tubes.

### Example 2

### Composition of a drop solution in the form of an emulsion (quantity for 1 000 millilitres)

| | |
|---|---|
| Roflumilast | 1.5 g |
| Medium chain length triglycerides | 100.0 g |
| Lecithin | 12.0 g |
| Glycerol | 25.0 g |
| Thiomersal | 0.1 g |
| Purified water | to 1 000 ml |

Production: First the roflumilast and then the lecithin are dissolved in the medium chain length triglycerides and the glycerol at 30°C-40°C. While stirring vigorously, the purified water is added and then homogenized until the droplet size of the disperse phase is below 500 nm. The thiomersal is dissolved by stirring. The emulsion is filtered through a 0.45 µm filter and dispensed into suitable containers.

### Example 3

Composition for a drop solution in form of an emulsion (quantity for 1000 millilitres)

| | |
|---|---|
| Roflumilast | 1,5 g |
| Lecithin | 1,5 g |
| Thiomersal | 0,1 g |
| Polyvidone (Koliidon^{®}17) | 10,0 g |
| 0,9% sodiumchloride solution to | 1000 ml |

While stirring vigorously the lecithin, thiomersal and polyvidone are dissolved in the 0,9% sodiumchloride solution. The micronized Roflumilast (90% of particles below 10µm) is stirred in and homogeneously dispersed.

### Production of tablets of the Invention

### Example A

### Weight based on a tablet containing 0.1 mg of roflumilast

| | | |
|---|---|---|
| | | |
| 1. | Roflumilast (micronized) | 0.100 mg |
| 2. | Lactose monohydrate | 49.660 mg |
| 3. | Corn starch | 13.390 mg |
| 4. | Polyvidone K90 | 1.300 mg |
| 5. | Magnesium stearate (vegetable) | 0.650 mg |
| | **Total** | **65.100 mg** |

Production: (1) is mixed with part of (3), and a trituration is produced in a planetary mill. The trituration is put together with (2) and the remaining amount of (3) in the product container of a fluidized bed granulation system, and a 5% granulation solution of (4) in purified water is sprayed on and dried under suitable conditions. (5) is added to the granules, and the mixture obtained after mixing is compressed in a tablet press to tablets having an average weight of 65.1 mg.

### Example B

### Weight based on a tablet containing 0.125 mg of roflumilast

| | | |
|---|---|---|
| 1. | Roflumilast | 0.125 mg |
| 2. | Lactose monohydrate | 49.660 mg |
| 3. | Corn starch | 13.390 mg |
| 4. | Polyvidone K90 | 1.300 mg |
| 5. | Magnesium stearate (vegetable) | 0.650 mg |
| | **Total** | **65.125 mg** |

Production: (1) is mixed with part of (3), and a trituration is produced in a planetary mill. The trituration is put together with (2) and the remaining amount of (3) in the product container of a fluidized bed granulation system, and a 5% granulation solution of (4) in purified water is sprayed on and dried under suitable conditions. (5) is added to the granules, and the mixture obtained after mixing is compressed in a tablet press to tablets having an average weight of 65.125 mg.

### Example C

### Weight based on a tablet containing 0.25 mg of roflumilast

| | | |
|---|---|---|
| 1. | Roflumilast | 0.250 mg |
| 2. | Microcrystalline cellulose | 33.900 mg |
| 3. | Corn starch | 2.500 mg |
| 4. | Polyvidone K90 | 2.250 mg |
| 5. | Sodium carboxymethylstarch (type A) | 20.000 mg |
| 6. | Magnesium stearate (vegetable) | 0.600 mg |
| | **Total** | **59.500 mg** |

Production: (1) is mixed with part of (3), and a trituration is produced in a planetary mill. The trituration is put together with (2), (5) and the remaining amount of (3) in the product container of a fluidized bed granulation system, and a 5% granulation solution of (4) in purified water is sprayed on and dried under suitable conditions. (6) is added to the granules, and the mixture obtained after mixing is compressed in a tablet press to tablets having an average weight of 59.5 mg.

### EXample D

### Weight based on a tablet containing 0.25 mg of roflumilast

| | | |
|---|---|---|
| 1. | Roflumilast | 0.250 mg |
| 2. | Lactose monohydrate | 49.660 mg |
| 3. | Corn starch | 13.390 mg |
| 4. | Polyvidone K90 | 1.300 mg |
| 5. | Magnesium stearate (vegetable) | 0.650 mg |
| | **Total** | **65.250 mg** |

Production: (1) is mixed with part of (3), and a trituration is produced in a planetary mill. The trituration is put together with (2) and the remaining amount of (3) in the product container of a fluidized bed granulation system, and a 5% granulation solution of (4) in purified water is sprayed on and dried under suitable conditions. (5) is added to the granules, and the mixture obtained after mixing is compressed in a tablet press to tablets having an average weight of 65.25 mg.

### Example E

### Weight based on a tablet containing 0.5 mg of roflumilast

| | | |
|---|---|---|
| 1. | Roflumilast | 0.500 mg |
| 2. | Lactose monohydrate | 49.660 mg |
| 3. | Corn starch | 13.390 mg |
| 4. | Polyvidone K90 | 1.300 mg |
| 5. | Magnesium stearate (vegetable) | 0.650 mg |
| | **Total** | **65.500 mg** |

Production: (1) is mixed with part of (3), and a trituration is produced in a planetary mill. The trituration is put together with (2) and the remaining amount of (3) in the product container of a fluidized bed granulation system, and a 5% granulation solution of (4) in purified water is sprayed on and dried under suitable conditions. (5) is added to the granules, and the mixture obtained after mixing is compressed in a tablet press to tablets having an average weight of 65.500 mg.

### Example F

### Weight based on a tablet containing 0.5 mg of roflumilast

| | | |
|---|---|---|
| 1. | Roflumilast | 0.500 mg |
| 2. | Lactose monohydrate | 99.320 mg |
| 3. | Corn starch | 26.780 mg |
| 4. | Polyvidone K90 | 2.600 mg |
| 5. | Magnesium stearate (vegetable) | 1.300 mg |
| | **Total** | **130.500 mg** |

Production: (1) is mixed with part of (3), and a trituration is produced in a planetary mill. The trituration is put together with (2) and the remaining amount of (3) in the product container of a fluidized bed granulation system, and a 5% granulation solution of (4) in purified water is sprayed on and dried under suitable conditions. (5) is added to the granules, and the mixture obtained after mixing is compressed in a tablet press to tablets having an average weight of 130.5 mg.

### Example G

### Weight based on a tablet containing 2.5 mg of roflumilast

| | | |
|---|---|---|
| 1. | Roflumilast | 2.500 mg |
| 2. | Microcrystalline cellulose | 33.900 mg |
| 3. | Corn starch | 2.500 mg |
| 4. | Polyvidone K90 | 2.250 mg |
| 5. | Sodium carboxymethylstarch (type A) | 20.000 mg |
| 6. | Magnesium stearate (vegetable) | 0.600 mg |
| | **Total** | **61.750 mg** |

Production: (1) is mixed with part of (3), and a trituration is produced in a planetary mill. The trituration is put together with (2), (5) and the remaining amount of (3) in the product container of a fluidized bed granulation system, and a 5% granulation solution of (4) in purified water is sprayed on and dried under suitable conditions. (6) is added to the granules, and the mixture obtained after mixing is compressed in a tablet press to tablets having an average weight of 61.75 mg.

### Example H

### Production of tablets containing 0.1 mg of roflumilast as active ingredient (weight for a batch of 70 000 tablets)

| | | |
|---|---|---|
| 1. | Roflumilast (micronized) | 7.000 g |
| 2. | Lactose monohydrate | 3476.200 g |
| 3. | Corn starch | 937.300 g |
| 4. | Polyvidone K90 | 91.000 g |
| 5. | Magnesium stearate (vegetable) | 45.500 g |
| | **Total** | **4557.000 g** |

Production: (1) is mixed with 70 g of (3), and a trituration is produced in a planetary mill. The trituration is put together with (2) and the remaining amount of (3) in the product container of a fluidized bed granulation system, and a 5% granulation solution of (4) in purified water is sprayed on. (Spraying pressure: 3 bar; product temperature: 28-33°C; air flow rate in the first third of the spraying process: 100 m³/h; air flow rate subsequently during the spraying process: 150 m³/h; inlet air temperature: 40-70°C; spraying rate: 30-40 g/min). After spraying is complete, drying is carried out until the product temperature reaches 34°C. The granules are passed through a stainless steel sieve with a mesh width of 0.8 mm, and the relative surface moisture is measured and adjusted to a value in the range 20-50%. (5) is added to the granules, and the mixture obtained after mixing is compressed in a tablet press to tablets having an average weight of 65.1 mg.

### Example I

### Production of tablets containing 0.25 mg of roflumilast as active ingredient (weight for a batch of 70 000 tablets)

| | | |
|---|---|---|
| 1. | Roflumilast (micronized) | 35.000 g |
| 2. | Lactose monohydrate | 3476.200 g |
| 3. | Corn starch | 937.300 g |
| 4. | Polyvidone K90 | 91.000 g |
| 5. | Magnesium stearate (vegetable) | 45.500 g |
| | **Total** | **4585.000 g** |

Production: 19.25 g of (1) are mixed with 192.5 g of (3), and a trituration is produced in a planetary mill. The trituration is put together with (2) and the remaining amount of (3) in the product container of a fluidized bed granulation system, and a 5% granulation solution of (4) in purified water is sprayed on. (Spraying pressure: 3 bar; product temperature: 28-33°C; air flow rate in the first third of the spraying process: 100 m³/h; air flow rate subsequently during the spraying process: 150 m³/h; inlet air temperature: 40-70°C; spraying rate: 30-40 g/min). After spraying is complete, drying is carried out until the product temperature reaches 34°C. The granules are passed through a stainless steel sieve with a mesh width of 0.8 mm, and the relative surface moisture is measured and adjusted to a value in the range 20-50%. (5) is added to the granules, and the mixture obtained after mixing is compressed in a tablet press to tablets having an average weight of 65.5 mg.

### Example J

### Production of tablets containing 0.1 mg of roflumilast as active ingredient (weight for a batch of 70 000 tablets)

| | | |
|---|---|---|
| 1. | Roflumilast (micronized) | 7.000 g |
| 2. | Lactose monohydrate | 3476.200 g |
| 3. | Corn starch | 937.300 g |
| 4. | Polyvidone K90 | 91.000 g |
| 5. | Magnesium stearate (vegetable) | 45.500 g |
| | **Total** | **4557.000 g** |

Production: (1) is homogeneously suspended in a granulation solution of (4) in purified water. (2) and (3) are put into the product container of a suitable fluidized bed granulation system and granulated with the granulation suspension described above, and then dried. (5) is added to the granules, and the mixture obtained after mixing is compressed in a tablet press to tablets having an average weight of 65.1 mg.

### Example K

### Production of tablets containing 0.25 mg of roflumilast as active ingredient (weight for a batch of 70 000 tablets)

| | | |
|---|---|---|
| 1. | Roflumilast (micronized) | 35.000 g |
| 2. | Lactose monohydrate | 3476.200 g |
| 3. | Corn starch | 937.300 g |
| 4. | Polyvidone K90 | 91.000 g |
| 5. | Magnesium stearate (vegetable) | 45.500 g |
| | **Total** | **4585.000 g** |

Production: (1) is homogeneously suspended in a granulation solution of (4) in purified water. (2) and (3) are put into the product container of a suitable fluidized bed granulation system and granulated with the granulation suspension described above, and then dried. (5) is added to the granules, and the mixture obtained after mixing is compressed in a tablet press to tablets having an average weight of 65.25 mg.

### Example L

### Weight based on a tablet containing 0.25 mg of roflumilast

| | | |
|---|---|---|
| 1. | Roflumilast | 0.250 mg |
| 2. | Lactose monohydrate | 49.660 mg |
| 3. | Potato starch | 10.000 mg |
| 4. | Corn starch | 3.590 mg |
| 5. | PVP 25 | 1.500 mg |
| 6. | Magnesium stearate (vegetable) | 0.650 mg |
| | **Total** | **65.650 mg** |

Production: A dispersion is produced from (4) and water, and (1) is homogeneously suspended therein. (5) is dissolved in water and added to the dispersion. (2) and (3) are granulated in a suitable fluidized bed granulation system with the dispersion under suitable conditions. (6) is added to this mixture, and the mixture obtained after mixing is compressed in a tablet press to tablets having an average weight of 65.650 mg.

### Example M

### Weight based on a tablet containing 0.25 mg of roflumilast

| | | |
|---|---|---|
| 1. | Roflumilast | 0.250 mg |
| 2. | Lactose monohydrate | 49.660 mg |
| 3. | Corn starch | 13.390 mg |
| 4. | Polyvidone K90 | 1.300 mg |
| 5. | Gelatin | 1.300 mg |
| 6. | Magnesium stearate (vegetable) | 0.650 mg |
| | **Total** | **66.550 mg** |

Production: (1) is mixed with part of (3), and a trituration is produced in a planetary mill. The trituration is put together with (2) and the remaining amount of (3) in the product container of a fluidized bed granulation system, and a 5% granulation solution of (4) and (5) in purified water is sprayed on and dried under suitable conditions. (6) is added to the granules, and the mixture obtained after mixing is compressed in a tablet press to tablets having an average weight of 66.55 mg.

### Example M1

### Formulation for pediatric use

### Weight based on a tablet containing 0.125 mg of roflumilast

| | | |
|---|---|---|
| 1. | Roflumilast | 0.125 mg |
| 2. | Lactose monohydrate | 49.660 mg |
| 3. | Corn starch | 13.390 mg |
| 4. | Polyvidone K90 | 1.300 mg |
| 5. | Mannit | 32.238 mg |
| 6. | Flavor (Tutti Frutti) | 0.329 mg |
| 7. | PVP (insoluble) | 12.895 mg |
| 5. | Magnesium stearate (vegetable) | 1.649 mg |
| | **Total** | **111.586 mg** |

The formulation is produced according to a process disclosed above.

### Industrial applicability

The pharmaceutical preparations of the invention can be employed for the treatment and prevention (prophylaxis) of dry eye syndrome (keratitis sicca).

In one embodiment of the invention the method is characterized that the administration takes place by systemic application of the pharmaceutical preparation. In this case the eye disorder is dry eye syndrome (keratitis sicca).

The pharmaceutical preparations of the invention comprise the active pharmaceutical ingredient in the dose customary for the treatment of the particular disease. The dosage of the active ingredient is of the order of magnitude customary for PDE inhibitors, it being possible to administer the daily dose in one or more dosage units. Customary dosages are disclosed for example in WO 95/01338. The normal dose on systemic therapy (oral) is between 0.001 and 3 mg per kilogram and day. Pharmaceutical preparations preferred according to the invention for topical administration contain from 0.005 mg to 5 mg of roflumilast, preferably from 0.01 mg to 2.5 mg, particularly preferably 0.1 mg to 0.5 mg of roflumilast per dosage unit. Examples of pharmaceutical preparations of the invention contain 0.01 mg, 0.1 mg, 0.125 mg, 0.25 mg and 0.5 mg of roflumilast per dosage unit.

## Claims

1. Use of a compound selected from the group consisting of roflumilast, salts of roflumilast, the N-oxide of the pyridine residue of roflumilast or salts thereof for the manufacture of a pharmaceutical preparation for the prevention or treatment of a disease of the eye, wherein the disease of the eye is dry eye syndrome (keratitis sicca).

2. Use according to claim 1 wherein roflumilast is a compound of the formula I 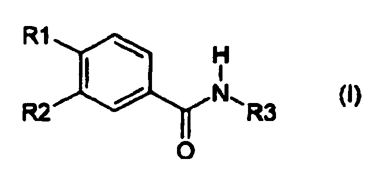 in which
R1 is difluoromethoxy,
R2 is cyclopropylmethoxy and
R3 is 3,5-dichloropyrid-4-yl.

3. Use according to claim 1, wherein the pharmaceutical preparation is an ophthalmological pharmaceutical preparation.

4. Use according to claim 3, wherein the pharmaceutical preparation is selected from the group of eyebaths, eye lotions, eye inserts, eye ointments, eye sprays, eye drops, preparations for intraocular application and eyelid ointments.

5. Use according to claim 1, wherein the pharmaceutical preparation is suitable for systemic application.

6. Use according to claim 4, wherein the pharmaceutical preparation is oily eye drops and comprises an excipient selected from the group of castor oil, peanut oil and medium chain length triglycerides.

7. Use according to claim 6, wherein medium chain length triglycerides are contained.

8. Ophthalmological Pharmaceutical preparation in the form of oily eye drops comprising a therapeutically effective and pharmacologically suitable amount of an active pharmaceutical ingredient selected from the group of compounds roflumi-last, salts of roflumilast, the N-oxide of roflumilast and salts together with one or more pharmaceutical acceptable carriers and/or excipients and which pharmaceutical preparation comprises medium chain length triglycerides.

9. The ophthalmological pharmaceutical preparation in the form of oily eye drops according to claim 8, wherein the active pharmaceutical ingredient is Roflumilast.

## Patentansprüche

1. Verwendung einer Verbindung, ausgewählt aus der Gruppe, bestehend aus Roflumilast, Salzen von Roflumilast, dem N-Oxid des Pyridinrests von Roflumilast oder Salzen davon, für die Herstellung eines pharmazeutischen Präparats zum Vorbeugen oder Behandeln einer Erkrankung des Auges, wobei die Erkrankung des Auges das Syndrom des trockenen Auges (Keratitis sicca) ist.

2. Verwendung gemäß Anspruch 1, wobei Roflumilast eine Verbindung der Formel I ist, 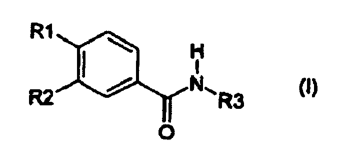 wobei
R1 Difluormethoxy ist,
R2 Cyclopropylmethoxy ist, und
R3 3,5-Dichlorpyrid-4-yl ist.

3. Verwendung gemäß Anspruch 1, wobei das pharmazeutische Präparat ein ophthalmologisches pharmazeutisches Präparat ist.

4. Verwendung gemäß Anspruch 3, wobei das pharmazeutische Präparat ausgewählt ist aus der Gruppe von Augenbädern, Augenlotionen, Augeneinsätzen, Augensalben, Augensprays, Augentropfen, Präparaten zur intraokulären Anwendung und Salben für die Augenlider.

5. Verwendung gemäß Anspruch 1, wobei das pharmazeutische Präparat für die systemische Anwendung geeignet ist.

6. Verwendung gemäß Anspruch 4, wobei es sich bei dem pharmazeutischen Präparat um ölige Augentropfen handelt und es einen Exzipienten umfasst, ausgewählt aus der Gruppe von Castoröl, Erdnussöl und Triglyceriden mit mittlerer Kettenlänge.

7. Verwendung gemäß Anspruch 6, wobei Triglyceride mit mittlerer Kettenlänge enthalten sind.

8. Opthalmologisches pharmazeutisches Präparat in der Form von öligen Augentropfen, umfassend eine therapeutisch wirksame und pharmakologisch geeignete Menge eines pharmazeutischen Wirkstoffs, ausgewählt aus der Gruppe der Verbindungen Roflumilast, Salzen von Roflumilast, dem N-Oxid von Roflumilast und Salzen, zusammen mit einem oder mehreren pharmazeutisch verträglichen Trägern und/oder Exzipienten, wobei das pharmazeutische Präparat Triglyceride mit mittlerer Kettenlänge umfasst.

9. Ophthalmologisches pharmazeutisches Präparat in der Form von öligen Augentropfen gemäß Anspruch 8, wobei der pharmazeutische Wirkstoff Roflumilast ist.

## Revendications

1. Utilisation d'un composé choisi dans le groupe constitué par le roflumilast, les sels du roflumilast, le N-oxyde du résidu de pyridine du roflumilast ou les sels de celui-ci pour la fabrication d'une préparation pharmaceutique destinée à la prévention ou au traitement d'une maladie de l'oeil, dans laquelle la maladie de l'oeil est le syndrome de l'oeil sec (la kératoconjonctivite sèche).

2. Utilisation selon la revendication 1 dans laquelle le roflumilast est un composé représenté par la formule I 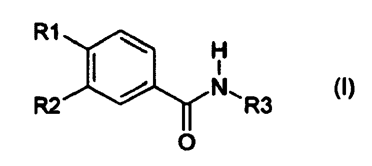 dans laquelle
R1 est un groupe difluorométhoxy,
R2 est un groupe cyclopropylméthoxy et
R3 est un groupe 3,5-dichloropyrid-4-yle.

3. Utilisation selon la revendication 1, dans laquelle la préparation pharmaceutique est une préparation pharmaceutique ophtalmologique.

4. Utilisation selon la revendication 3, dans laquelle la préparation pharmaceutique est choisie dans le groupe constitué par les bains pour les yeux, les lotions pour les yeux, les inserts pour les yeux, les onguents pour les yeux, les vaporisateurs ophtalmiques, les gouttes ophtalmiques, les préparations pour application intraoculaire et les onguents pour les paupières.

5. Utilisation selon la revendication 1, dans laquelle la préparation pharmaceutique est appropriée à une application systémique.

6. Utilisation selon la revendication 4, dans laquelle la préparation pharmaceutique consiste en des gouttes ophtalmiques huileuses et comprend un excipient choisi dans le groupe constitué par l'huile de ricin, l'huile d'arachide et les triglycérides ayant une longueur de chaîne moyenne.

7. Utilisation selon la revendication 6, dans laquelle des triglycérides ayant une longueur de chaîne moyenne sont contenus.

8. Préparation pharmaceutique ophtalmologique sous la forme de gouttes ophtalmiques huileuses comprenant une quantité thérapeutiquement efficace et pharmacologiquement appropriée d'un ingrédient pharmaceutique actif choisi dans le groupe de composés constitué par le roflumilast, les sels du roflumilast, le N-oxyde du roflumilast et des sels conjointement avec un ou plusieurs véhicules et/ou excipients pharmaceutiquement acceptables et laquelle préparation pharmaceutique comprend des triglycérides ayant une longueur de chaîne moyenne.

9. Préparation pharmaceutique ophtalmologique sous la forme de gouttes ophtalmiques huileuses selon la revendication 8, dans laquelle l'ingrédient pharmaceutique actif est le Roflumilast.
